# FASCICULE DE BREVET EUROPEEN

(11) **EP 1 953 218 B1**
(45) Date de publication et mention de la délivrance du brevet: **12.03.2014**
(21) Numéro de dépôt: 08290019.2
(22) Date de dépôt: 09.01.2008
(51) Int. Cl.: C12M 1/00

(54) **Récipient destiné à la culture de cellules, notamment des lymphocytes T**
Behälter für die Zellkultur, insbesondere von T-Lymphozyten
Container designed for cell cultures, in particular T lymphocytes

(30) Priorité: 12.01.2007 FR 0700252
(43) Date de publication de la demande: 06.08.2008
(73) Titulaire: Maco Pharma, 59400 Mouvaux (FR)
(72) Inventeur: Heron, Antoine, 59250 Halluin (FR)
(74) Mandataire: Sayettat, Julien Christian

(56) Documents cités:
- EP-A- 0 471 947
- WO-A-96/30497
- WO-A-98/07828
- FR-A1- 2 797 887

## Description

L'invention concerne un récipient destiné à la culture de cellules *in vitro.*

Le récipient est destiné à la culture de cellules en suspension et notamment à la culture *in vitro* des lymphocytes T, en particulier des lymphocytes T infiltrant les tumeurs (TIL).

Une technique prometteuse de traitement des tumeurs est l'immunothérapie adoptive. Selon cette technique, des cellules lymphoïdes sont prélevées d'un patient, stimulées et amplifiées *in vitro* avant d'être réinjectées au patient.

La stimulation est réalisée par adjonction d'interleukine 2 (IL 2) et/ou en présence de cellules nourricières allogéniques non proliférantes. Dans ce dernier cas, il est nécessaire de concentrer les cellules dans un espace confiné afin de favoriser leur communication.

C'est pourquoi, cette étape de stimulation est généralement réalisée dans des récipients de culture ouverts tels que des puits à fond rond.

Cependant, cette culture en système ouvert n'est pas compatible avec un usage clinique en routine. En effet, dans un cadre médical, les récipients de culture doivent présenter des garanties en matière de confinement des cellules, de prévention des risques de contamination et des erreurs techniques de manipulation. Lesdits récipients se doivent donc de respecter les règles strictes de bonne pratique des produits transfusables pour le conditionnement clos et le transfert des cellules.

Il existe des poches souples pour cultiver des cellules exploitées en thérapeutique humaine, notamment dans le cadre du développement des protocoles cliniques d'expansion ex vivo des cellules souches hématopoïétiques issues de prélèvement de moelle osseuse, de sang périphérique et de sang de cordon. Les documents US 6 297 046 et EP 471 947 illustrent de telles poches souples destinées à la culture de cellules.

Cependant, ces poches souples, posées à plat dans l'incubateur lors de la phase de culture, ne sont pas adaptées pour la co-culture de TIL avec des cellules nourricières allogéniques, puisqu'elles ne permettent pas de concentrer les cellules dans un espace confiné afin de favoriser leur communication.

Il convient de développer un récipient et un procédé de culture qui puissent respecter les bonnes pratiques, notamment en terme de confinement et de risque de contamination.

A cet effet, l'invention propose un récipient pour la mise en oeuvre d'une étape d'un procédé de culture in vitro de cellules, comprenant :
- une première et une deuxième feuilles en matériau thermoplastique souple solidarisées l'une à l'autre au voisinage de leur périphérie de sorte à former un volume intérieur ;
- une voie d'accès audit volume qui est agencée pour permettre l'introduction des cellules ;
- un cordon de soudure entre les feuilles qui est agencé pour former, dans le volume intérieur, deux compartiments communiquant entre eux et avec la voie d'accès, ledit cordon de soudure étant discontinu et étant associé de façon étanche à un bord du récipient.

D'autres objets et avantages de l'invention apparaîtront au cours de la description qui suit en référence aux dessins annexés dans lesquels :
- La figure 1 représente de façon schématique une vue de face du récipient selon un mode de réalisation,
- la figure 2 représente de façon schématique une vue de face du récipient selon un autre mode de réalisation de l'invention, tel qu'il est positionné pendant la stimulation des cellules.
- la figure 3 représente de façon schématique une coupe transversale du récipient de la figure 1.
- la figure 4 représente de façon schématique une vue de face du récipient selon un troisième mode de réalisation, le récipient contenant des cellules et un milieu de culture.
- la figure 5 représente de façon schématique un système clos comprenant un récipient d'émergence, un récipient de stimulation et un récipient d'amplification des lymphocytes T.

La culture de cellules consiste à faire croître des cellules hors de leur organisme ("ex-vivo") ou de leur milieu d'origine, dans un but thérapeutique, d'expérimentation scientifique ou de production biotechnologique.

Lors d'un procédé de culture de cellules, l'une des premières étapes consiste à isoler des cellules à partir d'un organisme vivant. Dans le cas des lymphocytes T infiltrant la tumeur (TIL), cette étape comprend l'émergence des lymphocytes T contenus dans une tumeur. Cette étape est réalisée par exemple, par immersion de fragments tumoraux de la tumeur dans un milieu de culture.

Le milieu de culture peut être un milieu de base disponible dans le commerce, tel que Dulbecco's Modified Eagle's Medium (DMEM), Minimal Essential Medium (MEM), CMRL, EMEM, RPMI1640, DMEM/F-10, DMEM/F12 ou un milieu de culture disponible dans le commerce tel que X-VIVO^{™}.

Ce milieu de culture peut être supplémenté avec divers additifs couramment utilisés par l'homme du métier. Parmi les additifs, on peut citer du sérum ou un substitut de sérum, des acides aminés non essentiels, la L-glutamine, des vitamines, des antibiotiques, le béta-mercaptoéthanol, des facteurs de croissance, des cytokines, etc.

Une autre étape d'un procédé de culture consiste à maintenir, faire proliférer et/ou amplifier les cellules isolées. Cette étape est généralement réalisée en mettant les cellules en suspension dans un milieu de culture, notamment différent de celui utilisé pour l'isolement des cellules.

Les cellules peuvent également subir différents traitements tels que la différentiation, l'activation ou la stimulation. Ces traitements sont réalisés généralement par adjonction dans le milieu de culture de substances ou de cellules spécifiques, selon le but recherché.

Dans le cas des lymphocytes T, notamment TIL, ceux-ci sont stimulés par exemple en présence de cellules nourricières allogéniques non proliférantes et d'interleukine 2.

Ces différentes étapes sont réalisées dans des récipients spécifiques au type de cellules et au type de traitement subi par les cellules.

L'invention propose un récipient pour la mise en oeuvre d'une étape d'un procédé de culture *in vitro* de cellules, notamment de cellules non adhérentes. En particulier, le récipient permet la culture de cellules provenant d'une ou plusieurs populations dans un espace confiné.

Par exemple, le récipient est utilisé pour la stimulation des lymphocytes infiltrant la tumeur (TIL ou Tumor Infiltrating Lymphocytes) par coculture avec d'autres cellules telle que les cellules nourricières allogéniques non proliférantes.

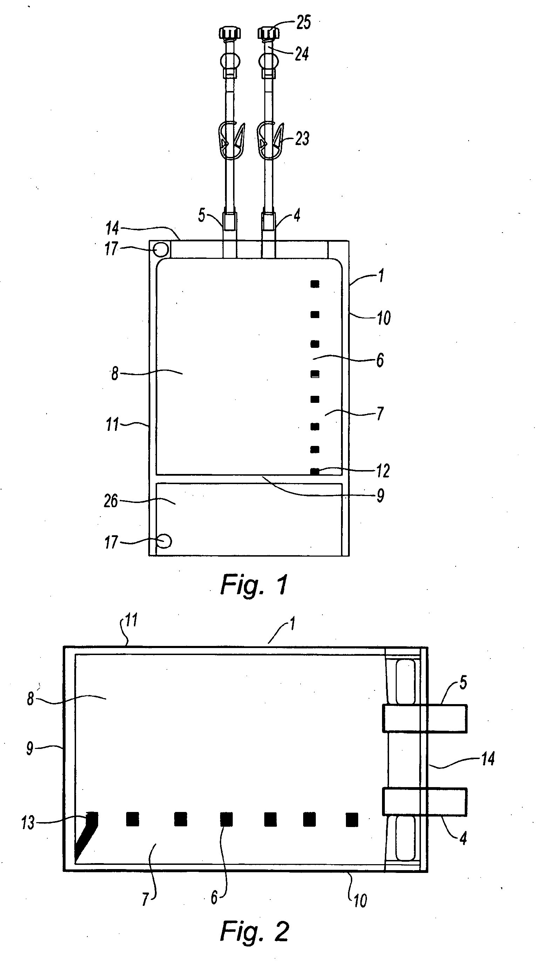

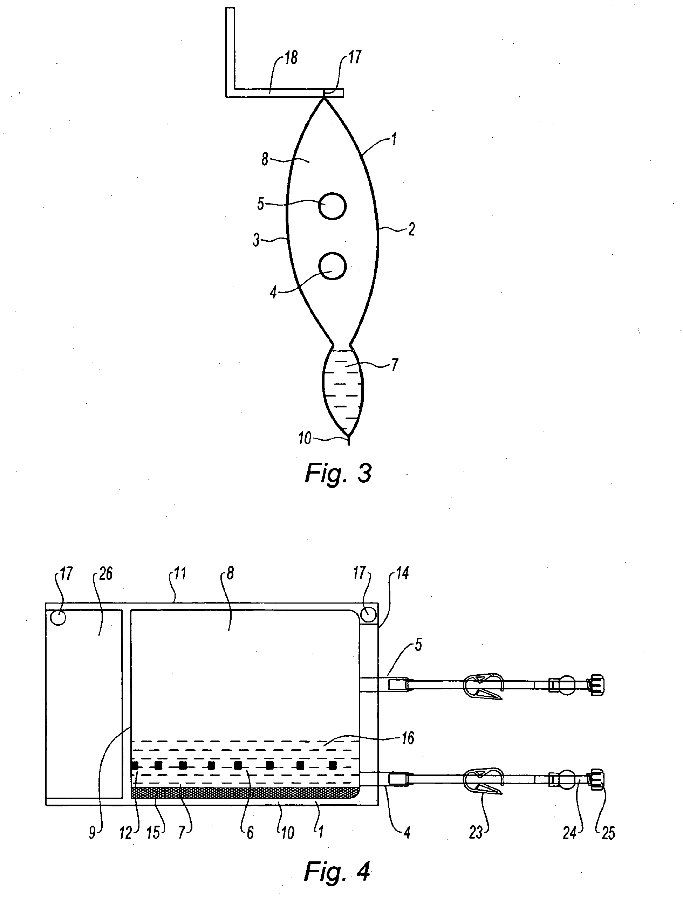

Selon les figures 1 et 3, le récipient 1 comprend une première et une deuxième feuilles 2,3 en matériau thermoplastique souple solidarisées l'une à l'autre au voisinage de leur périphérie de sorte à former un volume intérieur.

Des exemples de matériau thermoplastique souple sont le polychlorure de vinyle, l'alcool polyvinylique, le polyéthylène téréphtalate ou le polystyrène. D'autres exemples sont l'EVA, des polyoléfines tel que le polypropylène, le polyéthylène et/ou le polybutadiène, ou des polymères fluorés tel que l'éthylène-propylène fluoré (FEP), le Teflon® PFA ou le polytrétrafluoréthylène (PTFE). Ces derniers exemples de matériau présentent l'avantage d'être perméables aux gaz et stérilisables.

Selon une réalisation possible, les feuilles sont soudées. Toutefois, les feuilles peuvent également être solidarisées par une méthode différente, notamment par collage.

Le récipient comprend au moins une voie d'accès 4 au volume intérieur qui est agencée pour permettre l'introduction des cellules. Selon une variante, cette voie d'accès permet également la récupération des cellules.

Selon une autre variante, le récipient comprend deux voies d'accès 4,5 au volume intérieur, respectivement pour l'introduction et la récupération des cellules.

La voie d'accès 4,5 est par exemple formée d'une portion de tubulure, soudée entre les deux feuilles 2,3 formant le récipient. Dans une variante non représentée, la tubulure s'étend dans le volume intérieur du récipient.

Comme représentée sur la figure 1, la tubulure est munie d'un clamp 23 pour contrôler l'écoulement de fluide au travers de la tubulure. L'extrémité de la tubulure est munie d'un connecteur 24 de type Luer femelle, fermée par un bouchon 25 amovible.

Ce connecteur 24 permet de venir connecter une seringue remplie de cellules ou un container de milieu de culture.

En relation avec la figure 1 ou 2, le récipient comprend un cordon de soudure 6 entre les feuilles qui est agencé pour former, dans le volume intérieur, deux compartiments 7,8 communiquant entre eux et avec la voie d'accès 4, ledit cordon de soudure 6 étant discontinu et étant associé de façon étanche à un bord 9 du récipient.

Les deux compartiments 7,8 communiquent entre eux et avec la voie d'accès 4, de sorte qu'un fluide déversé à partir de la voie d'accès 4 peut s'écouler dans chacun des deux compartiments 7,8.

L'un des deux compartiments 7 du récipient 1, dit chambre de tranquillisation, formé entre l'un des bords 10 du récipient et le cordon de soudure 6, est destiné à recevoir les cellules à cultiver, notamment des cellules non adhérentes en suspension dans un peu de milieu de culture.

L'autre compartiment 8, dit chambre technique, formé entre le bord 11 opposé au bord délimitant la chambre de tranquillisation 7 et le cordon de soudure 6, est destiné à contenir au moins partiellement un milieu de culture. Notamment, la chambre technique 8 est utilisée pour introduire et/ou retirer du milieu de culture dans le volume intérieur du récipient 1, sans perturber les cellules placées dans la chambre de tranquillisation 7.

En effet, le cordon de soudure 6 ralentit et dévie un écoulement de fluide arrivant sur lui, de sorte que les cellules ne sont pas en contact direct avec le flux du milieu nouvellement introduit, limitant ainsi le déplacement ou la remise en suspension des cellules.

Lors de la phase de culture des cellules, le récipient 1 est positionné de sorte à disposer la chambre technique 8 au dessus de la chambre de tranquillisation 7. Dans cette position, les cellules sédimentent dans le fond de la chambre de tranquillisation 7 qui présente une forme en V comme illustré sur la figure 3, favorisant la concentration et la communication des cellules en culture.

Comme illustré sur les figures, le cordon de soudure 6 est discontinu, c'est-à-dire qu'il présente une pluralité d'interruptions, notamment plus de trois. Ces interruptions permettent un échange fluidique limité entre la chambre de tranquillisation 7 et la chambre technique 8.

Le cordon de soudure 6 est associé de façon étanche à un bord 9 du récipient, de sorte que la chambre technique 8 et la chambre de tranquillisation 7 ne communiquent pas entre elles à ce niveau. Ainsi, les cellules placées dans la chambre de tranquillisation 7 ne peuvent pas être transférées dans la chambre technique 8, à ce niveau. Notamment, lors de l'introduction des cellules, par exemple du côté opposée à cette zone, les cellules restent dans la chambre de tranquillisation 7.

La chambre de tranquillisation 7 fournit un espace confiné dans lequel les cellules sont tranquilles, c'est-à-dire peu ou pas déplacées après leur introduction dans le récipient 1.

Selon la figure 1, le cordon de soudure 6 est associé à un bord 9 du récipient par l'intermédiaire d'un des points de soudure 12 constituant le cordon. Le cordon est dans ce cas accolé au bord 9 du récipient 1.

Selon la figure 2, le point de soudure 13 le plus proche du bord 9 n'est pas accolé à celui-ci. Dans ce cas, ce point de soudure est étendu jusqu'à un bord 9 du récipient, notamment celui le plus proche.

Dans un mode de réalisation particulier, la voie d'accès 4 est prévue sur le bord 14 du récipient 1 qui est opposé au bord 9 auquel le cordon de soudure 6 est associé.

Lors de l'introduction des cellules dans la chambre de tranquillisation 7, les cellules ne peuvent pas remonter dans la chambre technique.

Selon un mode de réalisation, le récipient 1 comprend deux voies d'accès 4, 5 au volume intérieur, respectivement pour l'introduction et la récupération des cellules.

Comme illustré sur la figure 4, une voie d'accès 4,5 débouche dans chacun des compartiments 7,8.

La voie d'accès 4 qui débouche dans la chambre de tranquillisation 7 est utilisée pour l'introduction directement dans cette chambre des cellules mises en suspension dans un peu de milieu de culture.

La voie d'accès 5 qui débouche dans la chambre technique 8 est utilisée pour l'introduction de milieu de culture frais et/ou le retrait de milieu de culture usagé. Cette deuxième voie d'accès 5 permet de limiter la perturbation des cellules dans la chambre de tranquillisation 7 lors de ces étapes d'introduction et/ou retrait de milieu. Lors des changements de milieu, les turbulences au niveau des cellules sédimentées sont ainsi réduites.

En outre, avec deux voies d'accès 4,5, le risque de contamination lors des manipulations est réduit.

Dans une réalisation particulière, le cordon de soudure 6 s'étend parallèlement aux bords longitudinaux 10,11 dudit récipient.

Ainsi, lors de la phase de culture des cellules, lorsque le récipient 1 est positionné avec la chambre technique 8 au dessus de la chambre de tranquillisation 7 (fig. 4), les cellules sédimentent sur la plus grande longueur disponible du récipient 1.

En particulier, le cordon de soudure 6 est prévue à proximité d'un bord longitudinal 10 de sorte à former deux compartiments 7,8 de largeur différente.

Notamment, le compartiment 7 de plus petite largeur représente la chambre de tranquillisation. Le cordon de soudure 6, placé à proximité du bord du récipient 1, par exemple entre 1 et 3 cm du bord, notamment 2 cm du bord, crée et maintient un espace confiné pour une suspension de cellules, même après adjonction du milieu de culture.

Dans cette réalisation, les compartiments 7,8 communiquent transversalement entre eux, entre l'extrémité du cordon de soudure 6 et un bord transversal 14 dudit récipient.

Cette zone de communication transversale permet, lors de l'introduction de cellules par une voie d'accès débouchant dans la chambre technique, de déverser les cellules en suspension dans un milieu de culture directement dans la chambre de tranquillisation, sans rencontrer le cordon de soudure.

Selon une variante non représentée, la tubulure formant la voie d'accès 4 se prolonge dans le volume intérieur du récipient 1 jusque dans la chambre de tranquillisation 7.

Comme représenté sur les figures, le cordon de soudure 6 est discontinu et présente une pluralité d'interruptions, notamment au moins 3 interruptions.

Avantageusement, le cordon de soudure 6 est formé d'une succession de points de soudure qui sont régulièrement espacés.

Par exemple, les points de soudure ont une longueur comprise entre 1 mm et 5 mm, les espacements entre eux présentant une longueur comprise entre 1 mm et 1 cm.

La longueur des points de soudure est suffisante pour maintenir ensemble les deux feuilles formant le récipient et créer l'espace confiné. Les espacements entres les points de soudure doivent être de taille adaptée pour assurer un échange fluidique entre les deux compartiments 7,8.

On décrit maintenant un procédé de culture *in vitro* de cellules à l'aide du récipient de l'invention.

Selon ce procédé et en relation avec la figure 4, on introduit dans le récipient selon l'invention des cellules en suspension dans un milieu de culture 15 par l'intermédiaire de la voie d'accès 4, et on renouvelle le milieu de culture 16.

Le renouvellement du milieu de culture est réalisé par simple ajout par gravité de milieu de culture frais, par la voie d'accès utilisée pour l'introduction des cellules ou par une deuxième voie d'accès, s'il en existe une.

Par exemple, du milieu de culture est retiré du récipient avant l'ajout de milieu frais.

Selon ce procédé, on dispose le récipient 1 avec un compartiment au dessus de l'autre, de sorte à amener les cellules sur un bord du récipient.

Notamment, on dispose le récipient 1 avec la chambre technique 8 au dessus de la chambre de tranquillisation 7, de sorte à amener les cellules 15 sur le bord 10 du récipient 1.

Pour ce faire et comme illustré sur les figures 1 ou 4, le récipient est muni d'oeillets 17 placés à la périphérie du récipient 1, le long du bord 11 opposé au bord 10 délimitant la chambre de tranquillisation.

Lors de la phase de culture, on suspend le récipient 1 à l'aide de crochets 18 venant s'engager dans les oeillets (figure 3).

Selon une variante non représentée, le récipient est maintenu dans la position de culture par des pinces prévues sur un support.

Sur les figures 1 et 4, le récipient est muni d'un logement 26 pour glisser une étiquette d'identification.

On décrit maintenant plus en détail un procédé de culture *in vitro* de lymphocytes T, en particulier de lymphocytes T infiltrant les tumeurs dits TIL, modifiés ou non, à partir de prélèvement tumoral ou de sang issu de patient atteint de tumeur.

Les lymphocytes T infiltrant les tumeurs ou TIL sont des cellules immunes isolées à partir d'une tumeur ou de sang issu de patient atteint de tumeur. Réinjectés après stimulation, ils ont un pouvoir antitumoral, spécifique de la tumeur de laquelle ils sont issus.

Dans le cadre de la thérapie génique, ces lymphocytes T peuvent être modifiés par incorporation de gène tel qu'un gène de récepteurs chimères, augmentant leur pouvoir antitumoral.

Le procédé de culture des lymphocytes T comprend successivement au moins une étape d'émergence des lymphocytes T contenus dans la tumeur ou le sang prélevé, une étape de stimulation des lymphocytes T issus de l'étape d'émergence et une étape d'amplification des lymphocytes T stimulés.

Selon l'invention, l'étape de stimulation des lymphocytes T est réalisée dans un récipient de culture clos compartimenté.

Le récipient de culture compartimenté est un récipient adapté à la culture des lymphocytes T comprenant au moins deux compartiments communiquant l'un avec l'autre.

Un tel récipient est notamment souple et formé par assemblage de deux feuilles thermoplastiques souples solidarisées entre elles au voisinage de leur périphérie.

Un exemple de récipient de stimulation est décrit ci-dessus en relation avec les figures 1 à 4.

Le récipient est clos, c'est-à-dire que son volume intérieur n'est pas en contact avec l'air ambiant, notamment lors de la phase de culture, lorsque les cellules et le milieu de culture sont placés dans le récipient. Le récipient permet donc d'entretenir des cellules en culture dans un milieu adapté sans que celles-ci ne soient directement au contact avec l'environnement extérieur.

Selon le procédé de culture, on stimule les lymphocytes T en présence de cellules nourricières allogéniques non proliférantes.

Ces cellules nourricières allogéniques, c'est-à-dire provenant de la même espèce, mais d'un individu génétiquement différent, sont rendues non proliférantes par exemple par irradiation.

Notamment, on stimule les lymphocytes T par co-culture avec des cellules nourricières allogéniques non proliférantes consistant en un mélange formé de cellules mononucléées issues de sang de donneur humain sain et d'une lignée de cellules B transformées par l'EBV (Epstein-Barr Virus) de type LAZ.

Selon le procédé de culture, on co-cultive les cellules nourricières allogéniques non proliférantes et les lymphocytes T dans un milieu de culture sélectif de type X-VIVO 15^{™} supplémenté en interleukine 2.

Le milieu de culture X-VIVO 15^{™} est un milieu de culture sans sérum commercialisé par Cambrex Corp., Walkersville, Md., USA et optimisé pour la culture des TIL.

L'interleukine 2 joue un rôle important comme facteur de croissance et d'activation des lymphocytes.

Pour stimuler la croissance des TIL, au cours de l'étape de stimulation, on introduit dans le récipient de stimulation clos parallèlement aux lymphocytes, un agent mitotique tel que la PHA L (phytohémaglutinine de type L) apte à favoriser l'amplification des lymphocytes.

Comme décrit ci-dessus, la stimulation des lymphocytes T est réalisée dans un récipient 1 clos de stimulation comportant au moins deux compartiments 7,8, dits l'un chambre technique 8, l'autre chambre de tranquillisation 7, communiquant entre eux et au moins une voie d'accès 4 débouchant dans l'un des compartiments 7,8, dans lequel on introduit les lymphocytes et les cellules nourricières allogéniques non proliférantes en suspension dans un milieu de culture par l'intermédiaire de ladite voie d'accès 4.

Lors de l'étape de stimulation, on dispose le récipient 1 clos de stimulation avec la chambre technique 8 s'étendant au-dessus de la chambre de tranquillisation 7. Ainsi, les lymphocytes T et les cellules nourricières allogéniques non proliférantes sédimentent par gravité dans le fond de la chambre de tranquillisation.

Comme représenté sur la figure 3, ce fond, délimité par l'intersection des deux feuilles souples 2,3 formant le récipient 1, présente une forme en V adaptée au confinement des deux populations de cellules.

Pour maintenir la viabilité des lymphocytes T, on renouvelle le milieu de culture par l'intermédiaire de la ou d'une autre voie d'accès 4,5 du récipient 1 clos de stimulation.

Le renouvellement du milieu de culture est réalisé par ajout d'un milieu de culture frais, tel que le X-VIVO 15^{™} éventuellement supplémenté en IL2.

Selon la figure 1, le récipient clos 1 comporte au moins deux voies d'accès 4,5 débouchant dans la chambre technique 8. Dans ce cas, on introduit les lymphocytes et les cellules nourricières allogéniques non proliférantes en suspension dans un milieu de culture par l'intermédiaire d'une première voie d'accès 4 qui est positionnée la plus proche de la chambre de tranquillisation 7, et on renouvelle le milieu de culture par l'intermédiaire d'une seconde voie d'accès 5 qui est positionnée la plus éloignée de ladite chambre de tranquillisation.

L'introduction des cellules par la voie d'accès 4 la plus proche de la chambre de tranquillisation 7 permet de limiter le parcours des cellules dans le récipient 1, et donc de limiter leur stress.

L'utilisation de deux voies d'accès 4,5 différentes pour l'introduction des cellules et le renouvellement du milieu limite les risques de contamination lors de la manipulation du récipient.

Selon une variante du procédé de culture *in vitro* de lymphocytes T, on met en oeuvre l'étape d'émergence des lymphocytes T, en particulier de culture primaire du ou des prélèvements tumoraux, dans un récipient de culture clos.

L'étape d'émergence est par exemple réalisée par mise en culture de suspensions cellulaires issues de fragments de tumeurs ou du sang prélevé du patient, dans un milieu de culture sélectif comprenant de l'lL2. Après quelques jours de culture, seule persiste une population de lymphocytes T.

Le récipient de culture clos utilisé pour cette étape d'émergence est par exemple un récipient illustré sur les figures 1 à 4 ou un récipient similaire sans cordon de soudure.

Selon une autre variante du procédé, on transfère, après stimulation, les lymphocytes T stimulés dans un récipient de culture clos d'amplification.

Le récipient de culture clos d'amplification est par exemple un récipient illustré sur les figures 1 à 4 ou un récipient similaire sans cordon de soudure.

En particulier, on met en oeuvre les étapes d'émergence, de stimulation et d'amplification dans un récipient de culture clos différent d'une étape à une autre.

Notamment, le transfert d'un récipient clos à un autre est réalisé de façon stérile, par exemple en connectant à l'aide d'un appareil de connexion stérile de type SCD® de Terumo, les tubulures servant de voies d'accès.

En variante, les récipients clos d'émergence, de stimulation et d'amplification sont préconnectés entre eux au moment de la fabrication, de sorte à former un système clos.

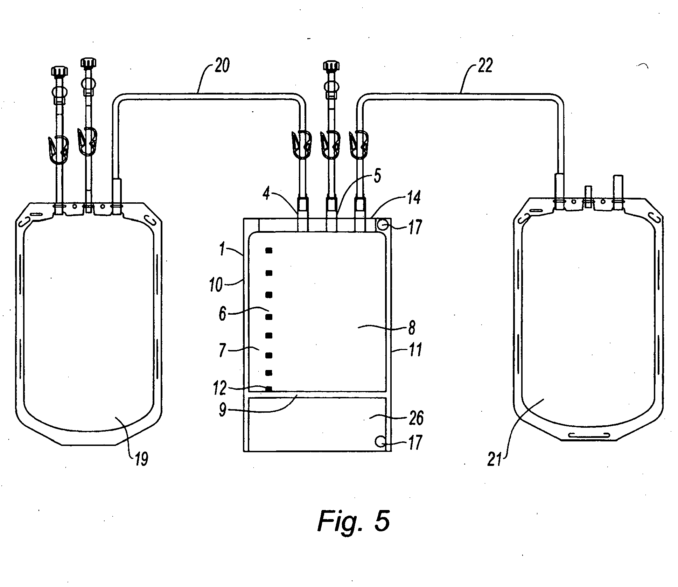

Un tel système est représenté sur la figure 5 et permet de réaliser la culture complète des lymphocytes T en système clos, limitant ainsi le risque de contamination.

Sur la figure 5, le récipient clos de stimulation 1 est connecté à un récipient d'émergence 19 par l'intermédiaire d'une tubulure 20, et à un récipient d'amplification 21 par l'intermédiaire d'une tubulure 22.

Les lymphocytes T, notamment TIL, ainsi stimulés et amplifiés par le procédé décrit, possèdent une activité cytotoxique utile pour le traitement des tumeurs chez un patient dans le cadre de l'immunothérapie adoptive.

## Revendications

1. Récipient (1) pour la mise en oeuvre d'une étape d'un procédé de culture in vitro de cellules, comprenant :
- une première et une deuxième feuilles (2,3) en matériau thermoplastique souple solidarisées l'une à l'autre au voisinage de leur périphérie de sorte à former un volume intérieur ;
- une voie d'accès (4) audit volume qui est agencée pour permettre l'introduction des cellules ;
- un cordon de soudure (6) entre les feuilles qui est agencé pour former, dans le volume intérieur, deux compartiments (7,8) communiquant entre eux et avec la voie d'accès (4), ledit cordon de soudure (6) étant discontinu et étant associé de façon étanche à un bord (9) du récipient (1).

2. Récipient (1) selon la revendication 1,
**caractérisé en ce que** la voie d'accès (4) est prévue sur le bord (14) du récipient qui est opposé au bord (9) auquel le cordon de soudure (6) est associé.

3. Récipient (1) selon la revendication 1 ou 2,
**caractérisé en ce qu'**il comprend deux voies d'accès (4,5) au volume intérieur, respectivement pour l'introduction et la récupération des cellules.

4. Récipient (1) selon la revendication 3,
**caractérisé en ce qu'**une voie d'accès (4,5) débouche dans chacun des compartiments (7,8).

5. Récipient selon l'une quelconque des revendications 1 à 4,
**caractérisé en ce que** les compartiments (7,8) sont formés respectivement entre un bord (10,11) dudit récipient et le cordon de soudure (6).

6. Récipient (1) selon la revendication 5,
**caractérisé en ce que** le cordon de soudure (6) s'étend parallèlement aux bords (10, 11) longitudinaux dudit récipient (1).

7. Récipient (1) selon la revendication 6,
**caractérisé en ce que** le cordon de soudure (6) est prévu à proximité d'un bord (10) longitudinal de sorte à former deux compartiments (7,8) de largeur différente.

8. Récipient (1) selon la revendication 6 ou 7,
**caractérisé en ce que** les compartiments (7,8) communiquent transversalement entre eux, entre l'extrémité du cordon de soudure (6) et un bord transversal (14) dudit récipient.

9. Récipient (1) selon l'une quelconque des revendications 1 à 8,
**caractérisé en ce que** le cordon de soudure (6) est formé d'une succession de points de soudure qui sont régulièrement espacés.

10. Récipient (1) selon la revendication 9,
**caractérisé en ce que** les points de soudure ont une longueur comprise entre 1 mm et 5 mm , les espacements entre eux présentant une longueur comprise entre 1 mm et 1 cm.

## Patentansprüche

1. Behälter (1) zur Durchführung eines Schritts eines Verfahrens zur in-vitro-Kultivierung von Zellen, umfassend:
- ein erstes und ein zweites Blatt (2, 3) aus einem flexiblen thermoplastischen Material, die miteinander in der Nähe ihres Umfangs fest verbunden sind, um ein inneres Volumen zu bilden;
- einen Zugangsweg (4) zu dem Volumen, der angeordnet ist, um die Einführung von Zellen zu ermöglichen;
- eine Schweißnaht (6) zwischen den Blättern, die angeordnet ist, um im inneren Volumen zwei Abteile (7, 8) zu bilden, die untereinander und mit dem Zugangsweg (4) kommunizieren, wobei die Schweißnaht (6) unterbrochen und auf dichte Weise mit einem Rand (9) des Behälters (1) verbunden ist.

2. Behälter (1) nach Anspruch 1,
**dadurch gekennzeichnet, dass** der Zugangsweg (4) auf dem Rand (14) des Behälters vorgesehen ist, der dem Rand (9) gegenüber liegt, mit dem die Schweißnaht (6) assoziiert ist.

3. Behälter (1) nach Anspruch 1 oder 2,
**dadurch gekennzeichnet, dass** er zwei Zugangswege (4, 5) zum inneren Volumen umfasst, jeweils für die Einführung und die Wiedergewinnung der Zellen.

4. Behälter (1) nach Anspruch 3,
**dadurch gekennzeichnet, dass** ein Zugangsweg (4, 5) in jedes der Abteile (7, 8) mündet.

5. Behälter nach einem der Ansprüche 1 bis 4, **dadurch gekennzeichnet, dass** die Abteile (7, 8) jeweils zwischen einem Rand (10, 11) des Behälters und der Schweißnaht (6) gebildet sind.

6. Behälter (1) nach Anspruch 5,
**dadurch gekennzeichnet, dass** sich die Schweißnaht (6) parallel zu den Längsrändern (10, 11) des Behälters (1) erstreckt.

7. Behälter (1) nach Anspruch 6,
**dadurch gekennzeichnet, dass** die Schweißnaht (6) in der Nähe eines Längsrands (10) vorgesehen ist, um zwei Abteile (7, 8) mit verschiedener Breite zu bilden.

8. Behälter (1) nach Anspruch 6 oder 7,
**dadurch gekennzeichnet, dass** die Abteile (7, 8) quer untereinander, zwischen dem Ende der Schweißnaht (6) und einem Querrand (14) des Behälters kommunizieren.

9. Behälter (1) nach einem der Ansprüche 1 bis 8, **dadurch gekennzeichnet, dass** die Schweißnaht (6) aus einer Aufeinanderfolge von Schweißpunkten gebildet ist, die regelmäßig beabstandet sind.

10. Behälter (1) nach Anspruch 9,
**dadurch gekennzeichnet, dass** die Schweißpunkte eine Länge aufweisen, die zwischen 1 mm und 5 mm liegt, wobei die Abstände zwischen ihnen eine Länge aufweisen, die zwischen 1 mm und 1 cm liegt.

## Claims

1. Container (1) for implementing a step of in vitro cell culture process, comprising:
- a first and a second sheets (2, 3) made of flexible thermoplastic made integral with one another in the vicinity of their periphery in such a way as to form an internal volume;
- an access path (4) to said volume that is arranged to allow for the introduction of cells;
- a weld seam (6) between the sheets that is arranged to form, in the internal volume, two compartments (7, 8) communicating with each other and with the access path (4), said weld seam (6) being discontinuous and being associated in a sealed manner with an edge (9) of the container (1).

2. Container (1) according to claim 1, **characterised in that** the access path (4) is provided on the edge (14) of the container which is opposite to the edge (9) to which the weld seam (6) is associated.

3. Container (1) according to claim 1 or 2, **characterised in that** it comprises two access paths (4, 5) to the internal volume, respectively for the introduction and the recovery of cells.

4. Container (1) according to claim 3, **characterised in that** an access path (4, 5) opens into each of the compartments (7, 8).

5. Container according to any of claims 1 to 4, **characterised in that** the compartments (7, 8) are formed respectively between an edge (10, 11) of said container and the weld seam (6).

6. Container (1) according to claim 5, **characterised in that** the weld seam (6) extends parallel to the longitudinal edges (10, 11) of said container (1).

7. Container (1) according to claim 6, **characterised in that** the weld seam (6) is provided in the vicinity of a longitudinal edge (10) in such a way as to form two compartments (7, 8) with a different length.

8. Container (1) according to claim 6 or 7, **characterised in that** the compartments (7, 8) communicate transversally with each other, between the end of the weld seam (6) and a transversal edge (14) of said container.

9. Container (1) according to any of claims 1 to 8, **characterised in that** the weld seam (6) is formed of a succession of regularly spaced weld points.

10. Container (1) according to claim 9, **characterised in that** the weld points have a length between 1 mm and 5 mm, with the spacing between them having a length between 1 mm and 1 cm.
